(19) Europäisches Patentamt
European Patent Office
Office européen des brevets

(11) **EP 4 406 643 A1**

(12) **EUROPEAN PATENT APPLICATION**
published in accordance with Art. 153(4) EPC

(43) Date of publication:
**31.07.2024 Bulletin 2024/31**

(21) Application number: **22872374.8**

(22) Date of filing: **02.02.2022**

(51) International Patent Classification (IPC):
**B01J 20/281** (2006.01)    **B01J 20/283** (2006.01)
**G01N 30/06** (2006.01)    **G01N 30/34** (2006.01)
**G01N 30/50** (2006.01)    **G01N 30/86** (2006.01)
**G01N 30/88** (2006.01)

(52) Cooperative Patent Classification (CPC):
**B01J 20/281; B01J 20/283; G01N 30/06;
G01N 30/34; G01N 30/50; G01N 30/86;
G01N 30/88**

(86) International application number:
**PCT/JP2022/004043**

(87) International publication number:
**WO 2023/047615 (30.03.2023 Gazette 2023/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.09.2021 JP 2021155278**

(71) Applicant: **Shimadzu Corporation
Nakagyo-ku,
Kyoto-shi,
Kyoto 604-8511 (JP)**

(72) Inventor: **IWATA, Natsuki
Kyoto-shi, Kyoto 604-8511 (JP)**

(74) Representative: **Müller-Boré & Partner
Patentanwälte PartG mbB
Friedenheimer Brücke 21
80639 München (DE)**

(54) **ANALYSIS METHOD**

(57)    An analytical method for performing, in parallel, a first analysis step of introducing a non-derivatized first sample 132 into a first column 44 together with a first mobile phase 54, and analyzing components contained in the first sample 132; and a second analysis step of introducing a derivatized second sample 137 into the second column 84 together with a second mobile phase 94, and analyzing components contained in the second sample 137, wherein the first sample 132 contains inosinic acid and guanylic acid, and in the first analysis step, by separating components contained in the first sample 132 using the first column 44 having a length of 185 mm or more, a chromatogram is obtained in which peaks corresponding to inosinic acid and guanylic acid are separated with a separation degree of 1.5 or more.

FIG.1

**Description**

TECHNICAL FIELD

The present invention relates to an analytical method.

BACKGROUND ART

For example, a liquid chromatograph disclosed in Patent Document 1 below is configured to be able to simultaneously execute analysis processing using a plurality of columns.

PRIOR ART DOCUMENT

PATENT DOCUMENT

Patent Document 1: WO 2020/183663 A

SUMMARY OF THE INVENTION

PROBLEMS TO BE SOLVED BY THE INVENTION

The inventor of the present application has studied performing analysis using the liquid chromatograph as described above in order to analyze both freshness and spoilage of food such as meat (including fish) in a short time. The freshness of food can be measured by analyzing ATP-related substances including inosinic acid. Meanwhile, food spoilage can be measured by analyzing histamine. When specific amino acids (hereinafter, it is referred to as "histamine/amino acid") including histamine and glutamic acid are analyzed, they are derivatized and then detected by a detector.

When both ATP-related substances and histamine/amino acids are analyzed, it is necessary to analyze ATP-related substances that are not derivatized and histamine/amino acids that are derivatized in different analysis systems. In this case, it is conceivable to prepare an analysis device for analyzing ATP-related substances and an analysis device for analyzing histamine and amino acids, but there arises a problem of installation space of the device and the like. Therefore, it is conceivable to analyze ATP-related substances on one of the two columns and to analyze histamine and amino acids on the other column using the liquid chromatograph as described above.

In the course of such studies, the inventors of the present application have attempted to analyze guanylic acid, which is an example of a flavor component, together with inosinic acid and guanylic acid contained in food simultaneously with an ATP-related substance and histamine/amino acid. In this case, it is conceivable to separately provide a column for analyzing guanylic acid and perform analysis on three columns, but it is efficient to perform analysis using the same column as the ATP-related substance that is not derivatized.

However, inosinic acid contained in ATP-related substances has a retention time close to that of guanylic acid. Therefore, it has been found that it is difficult to acquire a chromatogram in which peaks corresponding to inosinic acid and guanylic acid are separated.

The present invention has been made in view of the above circumstances, and an object thereof is to provide an analytical method capable of separating peaks corresponding to inosinic acid and guanylic acid contained in a first sample when analyzing components contained in each of a non-derivatized first sample and a derivatized second sample in parallel.

MEANS FOR SOLVING THE PROBLEMS

A first aspect of the present invention is an analytical method for performing, in parallel, a first analysis step of introducing a non-derivatized first sample into a first column together with a first mobile phase, and analyzing components contained in the first sample, and a second analysis step of introducing a derivatized second sample into a second column together with a second mobile phase, and analyzing components contained in the second sample, using the first column and the second column each constituted by an ODS column, wherein the first sample contains inosinic acid and guanylic acid, and in the first analysis step, by separating components contained in the first sample using the first column having a length of 185 mm or more, a chromatogram is obtained in which peaks corresponding to inosinic acid and guanylic acid are separated with a separation degree of 1.5 or more.

EFFECTS OF THE INVENTION

According to the present invention, when components contained in each of the non-derivatized first sample and the derivatized second sample are analyzed in parallel, peaks corresponding to inosinic acid and guanylic acid contained in the first sample can be separated.

BRIEF DESCRIPTION OF THE DRAWINGS

FIG. 1 is a block diagram illustrating an example of a configuration of a liquid chromatograph of the present embodiment.
FIG. 2 is a schematic view illustrating an example of a configuration of an autosampler of the present embodiment.
FIG. 3 is a block diagram illustrating an example of an electrical configuration of a liquid chromatograph of the present embodiment.
FIG. 4 is a schematic view for explaining a dispensing step of the present embodiment.
FIG. 5 is a schematic view for explaining the dispensing step of the present embodiment.
FIG. 6 is a schematic view for explaining the dispensing step of the present embodiment.
FIG. 7 is a schematic view for explaining the dispensing step of the present embodiment.
FIG. 8 is a schematic view for explaining a first analysis step and a second analysis step of the present embodiment.
FIG. 9 is a diagram illustrating a first chromatogram in a comparative example.
FIG. 10 is an enlarged view illustrating a part of the first chromatogram in FIG. 9.
FIG. 11 is a diagram illustrating the first chromatogram in examples.
FIG. 12 is a diagram illustrating another first chromatogram in examples.
FIG. 13 is a diagram illustrating an example of UV spectra in examples corresponding to FIGS. 11 and 12.
FIG. 14 is a block diagram specifically illustrating an electrical configuration of a liquid chromatograph of the present embodiment.

MODE FOR CARRYING OUT THE INVENTION

1. Configuration of liquid chromatograph

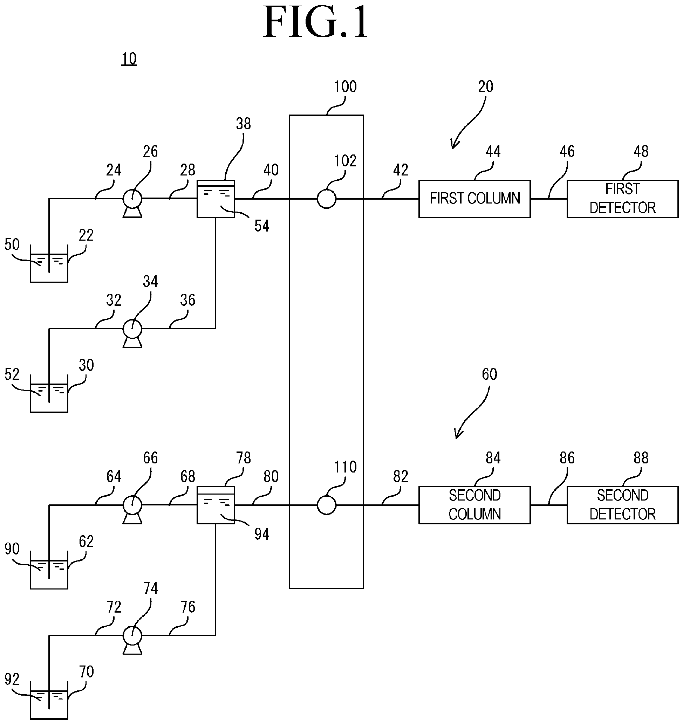

FIG. 1 is a block diagram illustrating an example of a configuration of a liquid chromatograph 10 of the present embodiment. As illustrated in FIG. 1, the liquid chromatograph 10 includes a first analysis system 20 and a second analysis system 60.

The first analysis system 20 includes a container 22, a piping 24, a first pump 26, a piping 28, a container 30, a piping 32, a second pump 34, a piping 36, a first mixer 38, a piping 40, a first switching valve 102, a piping 42, a first column 44, a piping 46, a first detector 48, and the like.

The second analysis system 60 includes a container 62, a piping 64, a third pump 66, a piping 68, a container 70, a piping 72, a fourth pump 74, a piping 76, a second mixer 78, a piping 80, a second switching valve 110, a piping 82, a second column 84, a piping 86, a second detector 88, and the like.

In the first analysis system 20, an upstream end of the piping 24 is inserted into the container 22, and a downstream end is connected to the first pump 26. The upstream end of the piping 28 is connected to the first pump 26, and the downstream end is connected to the first mixer 38.

The upstream end of the piping 32 is inserted into the container 30, and the downstream end is connected to the second pump 34. The upstream end of the piping 36 is connected to the second pump 34, and the downstream end is connected to the first mixer 38.

The upstream end of the piping 40 is connected to the first mixer 38, and the downstream end is connected to the first switching valve 102. The upstream end of the piping 42 is connected to the first switching valve 102, and the downstream end is connected to the first column 44. The upstream end of the piping 46 is connected to the first column 44, and the downstream end is connected to the first detector 48.

In addition, the first solution 50 is contained in the container 22, and the second solution 52 is contained in the container 30. Specifically, the first solution 50 and the second solution 52 are solvents constituting a mobile phase, and the second solution 52 has a higher content ratio of the organic solvent than that of the first solution 50.

In the second analysis system 60, the upstream end of the piping 64 is inserted into the container 62, and the downstream end is connected to the third pump 66. The upstream end of the piping 68 is connected to the third pump 66, and the downstream end is connected to the second mixer 78.

The upstream end of the piping 72 is inserted into the container 70, and the downstream end is connected to the fourth pump 74. The upstream end of the piping 76 is connected to the fourth pump 74, and the downstream end is connected to the second mixer 78.

The upstream end of the piping 80 is connected to the second mixer 78, and the downstream end is connected to the second switching valve 110. The upstream end of the piping 82 is connected to the second switching valve 110, and the downstream end is connected to the second column 84. The upstream end of the piping 86 is connected to the second column 84, and the downstream end is connected to the second detector 88.

In addition, a third solution 90 is contained in the container 62, and a fourth solution 92 is contained in the container 70. Specifically, the third solution 90 and the fourth solution 92 are solvents constituting the mobile phase, and the fourth solution 92 has a higher content ratio of the organic solvent than the third solution 90.

The first switching valve 102 and the second switching valve 110 constitute a part of an autosampler 100. The autosampler 100 is shared by the first analysis system 20 and the second analysis system 60.

In the first analysis system 20, for example, when the first pump 26 and the second pump 34 are driven, the first solution 50 and the second solution 52 are mixed by the first mixer 38 to generate the first mobile phase 54, and the first mobile phase 54 is led out from the first mixer 38 to the piping 40. That is, in this case, the first mobile phase 54 includes the first solution 50 and the second solution 52.

For example, when only the first pump 26 is driven, the second solution 52 is not introduced into the first mixer 38, so that the first mobile phase 54 containing only the first solution 50 is led out from the first mixer 38 to the piping 40.

Furthermore, when only the second pump 34 is driven, the first solution 50 is not introduced into the first mixer 38, so that the first mobile phase 54 containing only the second solution 52 is led out from the first mixer 38 to the piping 40.

Therefore, in the first analysis system 20, by appropriately driving the first pump 26 and the second pump 34 and adjusting the flow rates of the first solution 50 and the second solution 52, the mixing ratio of the first solution 50 and the second solution 52 in the first mobile phase 54 can be adjusted to perform gradient analysis.

In addition, a sample is injected into the first mobile phase 54 using the first switching valve 102 of the autosampler 100. Furthermore, the first mobile phase 54 into which the sample is injected is introduced into the first detector 48 after passing through the first column 44, whereby a chromatogram including peaks corresponding to respective components in the sample separated in the process of passing through the first column 44 is acquired.

In the second analysis system 60, similarly to the first analysis system 20, by appropriately driving the third pump 66 and the fourth pump 74, it is possible to perform gradient analysis by adjusting the mixing ratio of the third solution 90 and the fourth solution 92. The second mobile phase 94 in which the mixing ratio of the third solution 90 and the fourth solution 92 is adjusted is led out from the second mixer 78 to the piping 80.

In addition, a sample is injected into the second mobile phase 94 using the second switching valve 110 of the autosampler 100. Furthermore, the second mobile phase 94 into which the sample is injected is introduced into the second detector 88 after passing through the second column 84, whereby a chromatogram including peaks corresponding to the respective components in the sample separated in the process of passing through the second column 84 is acquired.

2. Configuration of autosampler

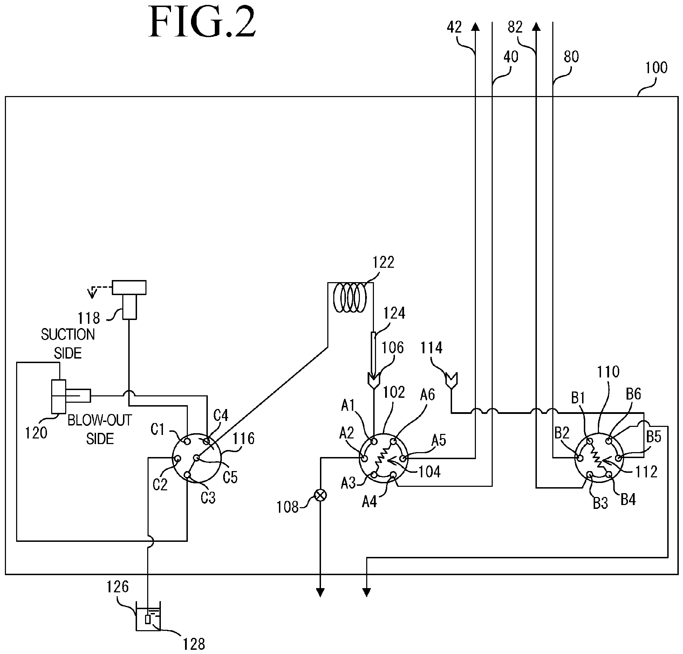

FIG. 2 is a schematic view illustrating an example of a configuration of the autosampler 100 of the present embodiment. As illustrated in FIG. 2, in addition to the first switching valve 102 and the second switching valve 110 described above, the autosampler 100 includes a first sample loop 104, a first injection port 106, an on-off valve 108, a second sample loop 112, a second injection port 114, a third switching valve 116, a rinsing port 118, a metering pump 120, a needle loop 122, a needle 124, and the like.

The first switching valve 102 is a six-way valve having six ports A1 to A6. The port A1 is connected to the first injection port 106. The port A2 is connected to the on-off valve 108. The port A3 is connected to the port A6 via the first sample loop 104. The port A4 is connected to the piping 40. The port A5 is connected to the piping 42.

Further, the rotor seal (not illustrated) of the first switching valve 102 has three passages that connect adjacent ports to each other. The first switching valve 102 is switched between the first state and the second state by the rotation of the rotor seal. The first state and the second state are different from each other in a combination of ports connected to each other.

When the first switching valve 102 is in the first state, as illustrated in FIG. 2, the port A1 and the port A2 are connected, the port A3 and the port A4 are connected, and the port A5 and the port A6 are connected.

That is, when the first switching valve 102 is in the first state, the piping 40 is connected to the piping 42 via the port A4, the port A3, the first sample loop 104, the port A6, the port A5, and the like. The first injection port 106 is connected to the on-off valve 108 via the port A1, the port A2, and the like.

On the other hand, when the first switching valve 102 is in the second state, the port A2 and the port A3 are connected, the port A4 and the port A5 are connected, and the port A6 and the port A1 are connected.

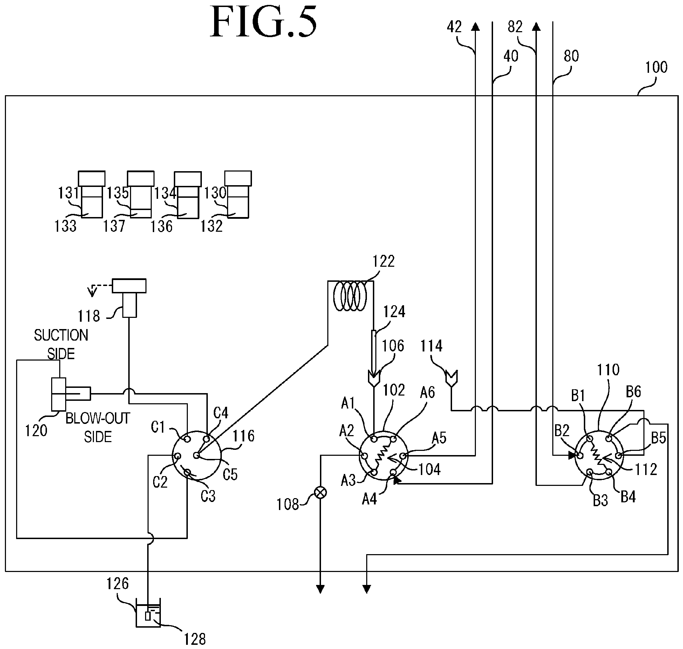

That is, when the first switching valve 102 is in the second state (refer to FIG. 5), the piping 40 is connected to the piping 42 via the port A4, the port A5, and the like. In addition, the first injection port 106 is connected to the on-off valve 108 via the portAl, the port A6, the first sample loop 104, the port A3, the port A2, and the like.

The second switching valve 110 is a six-way valve having six ports B1 to B6. The port B1 is connected to the port B4 via the second sample loop 112. The port B2 is connected to the piping 80. The port B3 is connected to the piping 82. The port B5 is connected to the second injection port 114. The port B6 is connected to the drainage passage.

Further, the rotor seal (not illustrated) of the second switching valve 110 has three passages connecting adjacent ports to each other, and is switched between the first state and the second state by the rotation of the rotor seal, similarly to the first switching valve 102.

When the second switching valve 110 is in the first state, as illustrated in FIG. 2, the port B1 and the port B2 are connected, the port B3 and the port B4 are connected, and the port B5 and the port B6 are connected.

That is, when the second switching valve 110 is in the first state, the piping 80 is connected to the piping 82 via the port B2, the port B1, the second sample loop 112, the port B4, the port B3, and the like. The second injection port 114 is connected to the drainage passage via the port B5, the port B6, and the like.

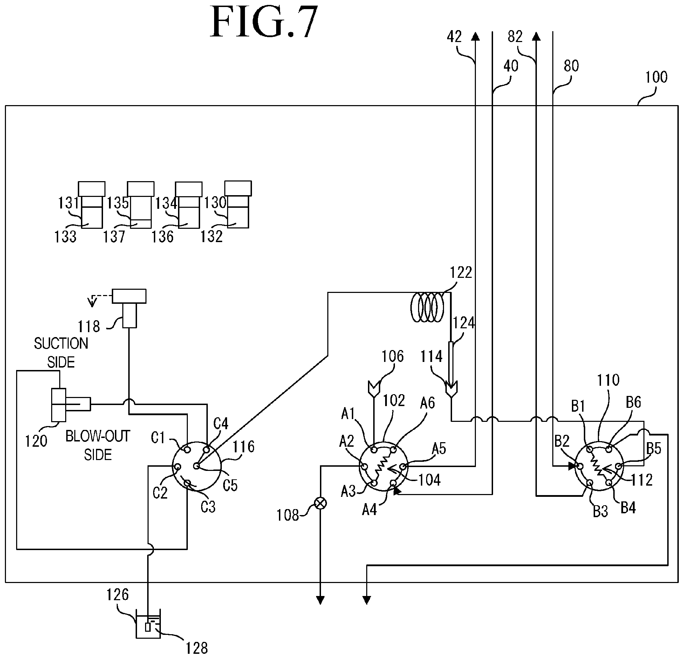

On the other hand, when the second switching valve 110 is in the second state (refer to FIG. 7), the port B2 and the port B3 are connected, the port B4 and the port B5 are connected, and the port B6 and the port B1 are connected.

That is, when the second switching valve 110 is in the second state, the piping 80 is connected to the piping 82 via the port B2, the port B3, and the like. In addition, the second injection port 114 is connected to the drainage passage via the port B5, the port B4, the second sample loop 112, the port B1, the port B6, and the like.

The third switching valve 116 is a rotary valve having five ports C1 to C5. The port C 1 is connected to the rinsing port 118. The port C2 is disposed outside the autosampler 100 and connected to a container 126 that contains the washing solution 128. The port C3 is connected to the suction side of the metering pump 120. The port C4 is connected to the blow-out side of the metering pump 120. The port C5 is connected to the needle 124 via the needle loop 122.

The rotor seal (not illustrated) of the third switching valve 116 has a passage that connects any one of the port C1, the port C2, the port C3, and the port C4 to the port C5, and a passage that connects adjacent ports to each other.

The third switching valve 116 can be switched to the first state, the second state, the third state, the fourth state, the fifth state, or the sixth state by the rotation of the rotor seal, and the combination of the ports connected to each other is different in each of the first state to the sixth state.

When the third switching valve 116 is in the first state, the port C3 and the port C5 are connected as illustrated in FIG. 2. That is, when the third switching valve 116 is in the first state, the suction side of the metering pump 120 is connected to the needle 124 via the port C3, the port C5, the needle loop 122, and the like.

When the third switching valve 116 is in the second state, the port C4 and the port C1 are connected. That is, when the third switching valve 116 is in the second state, the blow-out side of the metering pump 120 is connected to the rinsing port 118 via the port C4, the port C 1, and the like.

When the third switching valve 116 is in the third state, the port C2 and the port C3 are connected. That is, when the third switching valve 116 is in the third state, the suction side of the metering pump 120 is connected to the container 126 via the port C3, the port C2, and the like.

When the third switching valve 116 is in the fourth state (refer to FIG. 5), the port C4 and the port C5 are connected. That is, when the third switching valve 116 is in the fourth state, the blow-out side of the metering pump 120 is connected to the needle 124 via the port C4, the port C5, the needle loop 122, and the like.

When the third switching valve 116 is in the fifth state, the port C 1 and the port C5 are connected. That is, when the third switching valve 116 is in the fifth state, the rinsing port 118 is connected to the needle 124 via the port C 1, the port C5, the needle loop 122, and the like.

When the third switching valve 116 is in the sixth state, the port C2 and the port C5 are connected. That is, when the third switching valve 116 is in the sixth state, the container 126 is connected to the needle 124 via the port C2, the port C5, the needle loop 122, and the like.

Although not illustrated, the autosampler 100 movably supports the needle 124 and includes an electrically controllable moving mechanism. The first switching valve 102, the on-off valve 108, the second switching valve 110, the third switching valve 116, the metering pump 120, and the like can be electrically controlled.

3. Electrical configuration of liquid chromatograph

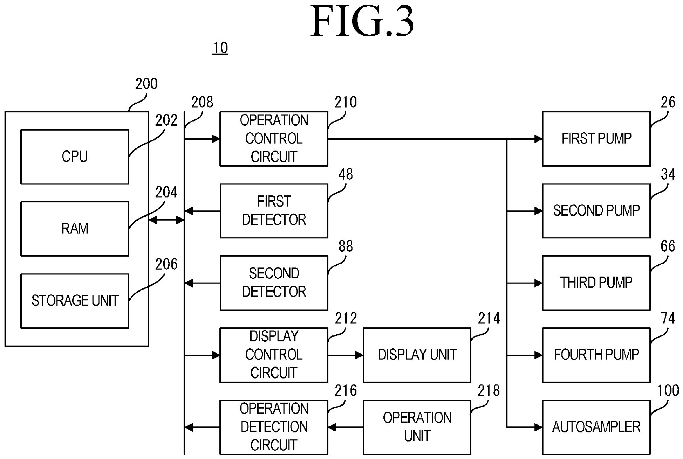

FIG. 3 is a block diagram illustrating an example of an electrical configuration of the liquid chromatograph 10 of the present embodiment. As illustrated in FIG. 3, the liquid chromatograph 10 includes a control unit 200, an operation control circuit 210, a display control circuit 212, a display unit 214, an operation detection circuit 216, an operation unit 218, and the like in addition to the first pump 26, the second pump 34, the first detector 48, the third pump 66, the fourth pump 74, the second detector 88, the autosampler 100, and the like.

In addition, each of the first detector 48, the second detector 88, the control unit 200, the operation control circuit 210, the display control circuit 212, and the operation detection circuit 216 is electrically connected to each other via a wiring 208 such as a bus.

Further, the operation control circuit 210 is electrically connected to the first pump 26, the second pump 34, the third pump 66, the fourth pump 74, and the autosampler 100. Furthermore, the display control circuit 212 is electrically connected to the display unit 214, and the operation detection circuit 216 is electrically connected to the operation unit 218.

The control unit 200 is responsible for overall control of the liquid chromatograph 10. The control unit 200 includes a central processing unit (CPU) 202. In addition, the control unit 200 includes a random access memory (RAM) 204 and a storage unit 206 that can be directly accessed by the CPU 202.

The CPU 202 controls each component of the liquid chromatograph 10. RAM 204 is used as a work area and a buffer area of the CPU 202. The storage unit 206 is a nonvolatile memory, and for example, a hard disc drive (HDD), a solid state drive (SSD), or the like is used as the storage unit 206.

The storage unit 206 stores a control program for controlling each component of the liquid chromatograph 10, execution data required for executing the control program, and the like. Note that the storage unit 206 may be configured to include the RAM 204.

The operation control circuit 210 generates a voltage for driving the first pump 26, the second pump 34, the third pump 66, the fourth pump 74, the autosampler 100, and the like, and appropriately applies the voltage thereto.

The display control circuit 212 includes a graphics processing unit (GPU), a video random access memory (VRAM), and the like, and the GPU generates a display image for displaying various screens on the display unit 214 in the VRAM under an instruction of the CPU 202 and outputs the generated display image to the display unit 214.

The display unit 214 is a general-purpose display device such as a liquid crystal display (LCD) or an electro-luminescence (EL) display.

The operation detection circuit 216 outputs a signal or data corresponding to the operation of the operation unit 218 to the control unit 200, specifically, the CPU 202.

The operation unit 218 includes hardware keys (operation keys). Examples of the operation key include a power key and the like. In addition, the operation unit 218 includes an input device. Examples of the input device include a keyboard and a mouse.

Furthermore, the input device may include a touch panel. In this case, the touch panel is provided on the display surface of the display unit 214. Further, the touch panel and the display unit 214 may be integrally formed.

4. Operation of liquid chromatograph

In such a liquid chromatograph 10, the dispensing step can be performed at an optional timing. For example, when a predetermined operation on the operation unit 218 is detected, the dispensing step is performed.

The dispensing step is a step of suctioning and discharging the sample. In the present embodiment, during the dispensing step, the first mobile phase 54 is introduced into the first column 44 and the second mobile phase 94 is introduced into the second column 84.

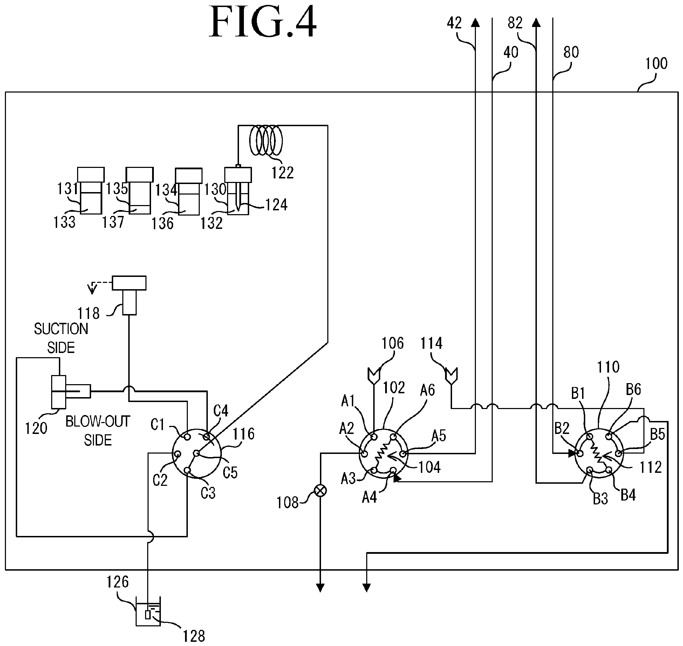

FIGS. 4 to 7 are schematic views for explaining a dispensing step of the present embodiment. As illustrated in FIGS. 4 to 7, a container 130 for containing a first sample 132 and a container 134 for containing a second sample 136 are set in advance in the autosampler 100.

In the present embodiment, the second sample 136 is derivatized using a derivatization reagent 133 contained in the container 131. Specifically, the derivatization reagent 133 is injected from the container 131 into the empty container 135, and the second sample 136 is injected from the container 134, whereby the derivatized second sample 137 is contained in the container 135. An example of derivatization includes fluorescent derivatization. The type of the derivatization reagent 133 is not particularly limited, and may be a mixture of a plurality of types (for example, two types) of derivatization reagents, or may be one type of derivatization reagent. On the other hand, the first sample 132 is not derivatized.

After the derivatization of the second sample 136 is started as described above, the first suction operation for suctioning the first sample 132 is performed. When the first suction operation is performed, the needle 124 is inserted into the container 130 as illustrated in FIG. 4. When the first suction operation is performed, the first switching valve 102, the second switching valve 110, and the third switching valve 116 are in the first state.

In the first suction operation, the metering pump 120 is controlled, and the first sample 132 is introduced into the needle loop 122 via the needle 124. The first sample 132 introduced into the needle loop 122 is stored in the needle loop 122.

After the first suction operation is performed, a first discharge operation for discharging the first sample 132 is performed. When the first discharge operation is performed, the needle 124 is inserted into the first injection port 106 as illustrated in FIG. 5. When the first discharge operation is performed, the first switching valve 102 is switched from the first state to the second state, and the third switching valve 116 is switched from the first state to the fourth state. Further, when the first discharge operation is performed, the on-off valve 108 is opened.

In the first discharge operation, the metering pump 120 is controlled, and the first sample 132 in the needle loop 122 is introduced into the first sample loop 104 via the first injection port 106. When the first discharge operation is performed, the on-off valve 108 is closed so that the first sample 132 in the first sample loop 104 does not flow out. The first sample 132 introduced into the first sample loop 104 is stored in the first sample loop 104.

After the first discharge operation is performed, a washing operation for washing the needle 124 is performed. Although not illustrated, when the washing operation is started, the third switching valve 116 is switched from the fourth state to the third state, so that the suction side of the metering pump 120 is connected to the container 126 via the port C3, the port C2, and the like, and the washing solution 128 in the container 126 is suctioned.

When the washing solution 128 in the container 126 is suctioned, the third switching valve 116 is switched from the third state to the second state, so that the blow-out side of the metering pump 120 is connected to the rinsing port 118 via the port C4, the port C 1, and the like, and the washing solution 128 is introduced into the rinsing port 118.

Further, when the washing solution 128 is introduced into the rinsing port 118, the needle 124 is inserted into the rinsing port 118, and the needle 124 is cleaned. In the present embodiment, since the first suction operation, the first discharge operation, and the washing operation as described above are performed after the derivatization reagent 133 and the second sample 136 are injected into the empty container 135, the derivatization of the second sample 136 can be efficiently performed using the time.

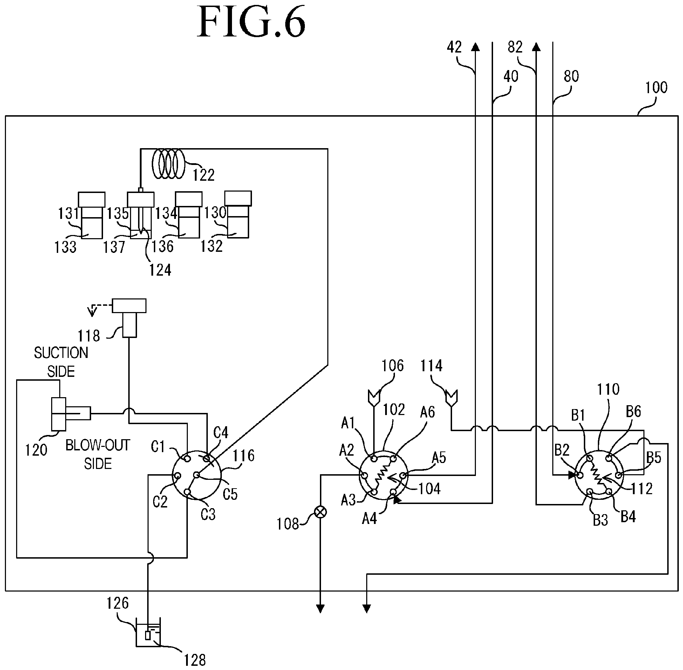

When the washing operation is performed in this manner, next, a second suction operation for suctioning the derivatized second sample 137 is performed. When the second suction operation is performed, the needle 124 is inserted into the container 135 as illustrated in FIG. 6. When the second suction operation is performed, the third switching valve 116 is switched from the second state to the first state.

In the second suction operation, the metering pump 120 is controlled, and the derivatized second sample 137 is introduced into the needle loop 122 via the needle 124. The derivatized second sample 137 introduced into the needle loop 122 is stored in the needle loop 122.

After the second suction operation is performed, a second discharge operation for discharging the derivatized second sample 137 is performed. When the second discharge operation is performed, the needle 124 is inserted into the second injection port 114 as illustrated in FIG. 7. When the second discharge operation is performed, the second switching valve 110 is switched from the first state to the second state, and the third switching valve 116 is switched from the first state to the fourth state.

In the second discharge operation, the metering pump 120 is controlled to introduce the derivatized second sample 137 in the needle loop 122 into the second sample loop 112 via the second injection port 114. At this time, the drainage passage is connected to the blow-out side of the metering pump 120 via the port B6, the port C4, and the like, and the port C3 connected to the suction side of the metering pump 120 is sealed. As a result, the derivatized second sample 137 introduced into the second sample loop 112 is stored in the second sample loop 112.

In the present embodiment, after the dispensing step is performed in this manner, the first analysis step and the second analysis step are performed in parallel. The first analysis step is a step of introducing the first sample 132 into the first column 44 together with the first mobile phase 54 and analyzing components contained in the first sample 132. The second analysis step is a step of introducing the derivatized second sample 137 into the second column 84 together with the second mobile phase 94 and analyzing components contained in the second sample 137. Note that "parallel" is not limited to a case where the first analysis step and the second analysis step are simultaneously started, and is a concept including a case where at least a part of the first analysis step and the second analysis step is simultaneously performed. Therefore, at least one of the start timing and the end timing of the first analysis step and the second analysis step may not be simultaneous.

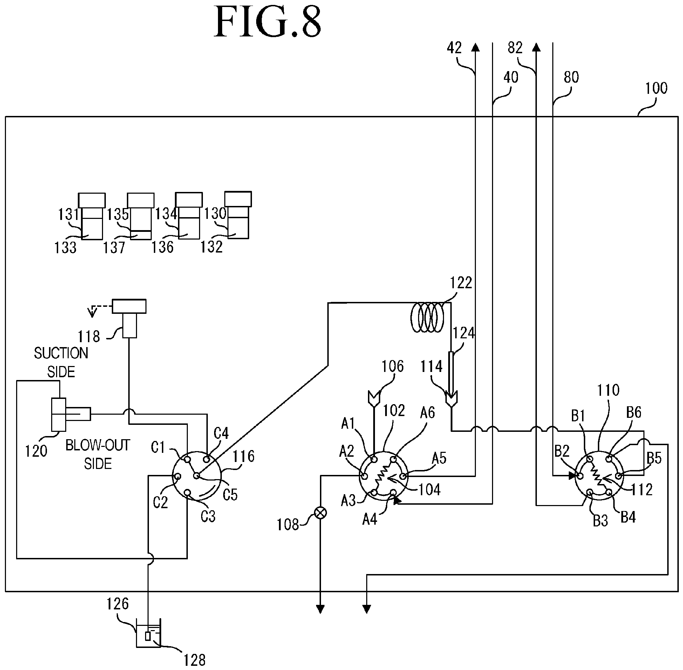

FIG. 8 is a schematic view for explaining the first analysis step and the second analysis step of the present embodiment. When the first analysis step and the second analysis step are performed, as illustrated in FIG. 8, the first switching valve 102 and the second switching valve 110 are switched from the second state to the first state.

When the first switching valve 102 is switched from the second state to the first state, the first sample 132 in the first sample loop 104 is introduced into the first mobile phase 54 introduced from the piping 40. Therefore, the first sample 132 is introduced into the first column 44 together with the first mobile phase 54.

When the second switching valve 110 is switched from the second state to the first state, the derivatized second sample 137 in the second sample loop 112 is introduced into the second mobile phase 94 introduced from the piping 80. Therefore, the derivatized second sample 137 is introduced into the second column 84 together with the second mobile phase 94.

When the first sample 132 passes through the first column 44, components in the first sample 132 are separated, and the separated components are detected by the first detector 48. When each component is detected by the first detector 48, the first chromatogram is acquired based on the detection result.

On the other hand, when the derivatized second sample 137 passes through the second column 84, each component in the second sample 137 is separated, and each separated component is detected by the second detector 88. When each component is detected by the second detector 88, the second chromatogram is acquired based on the detection result.

The introduction of the first mobile phase 54 into the first column 44 and the introduction of the second mobile phase 94 into the second column 84 are continued until both the first analysis step and the second analysis step are completed. In addition, the separation degree of each component in the first chromatogram changes according to the dimension and type of the first column 44, and the separation degree of each component in the second chromatogram changes according to the dimension and type of the second column 84.

Furthermore, the configuration of the autosampler 100 is not particularly limited as long as the first analysis step and the second analysis step can be performed in parallel as described above. For example, the first sample 132 may be introduced into the first column 44 along the total volume injection method, and the derivatized second sample 137 may be introduced into the second column 84 along the loop injection method.

4. Example

Hereinafter, an example in which the first sample 132 and the second sample 136 are analyzed using the liquid chromatograph 10 as described above will be described. In this example, analysis conditions in a case where both freshness and spoilage of food such as meat (including fish) are analyzed by one liquid chromatograph 10, and a flavor component contained in the food is also simultaneously analyzed by the liquid chromatograph 10 were examined.

The freshness of food can be measured by analyzing ATP-related substances. The ATP-related substances include nucleic acid-related substances such as hypoxanthine (Hx), inosinic acid (IMP), inosine (HxR), adenosine monophosphate (AMP), adenosine diphosphate (ADP), and adenosine triphosphate (ATP). The first sample 132 contains at least inosinic acid (IMP) among the above-described six components of ATP-related substances. In addition, the first sample 132 contains guanylic acid (GMP), which is an example of a flavor component contained in food, in addition to the ATP-related substances. The first sample 132 is subjected to pretreatment.

Food spoilage can be measured by analyzing histamine (Him). Other analytes may include specific amino acids known as nutritional components (including flavor components). Specifically, examples of the specific amino acid include aspartic acid (Asp), glutamic acid (Glu), asparagine (Asn), serine (Ser), glutamine (Gln), glycine (Gly), histidine (His), threonine (Thr), β-alanine (β-Ala), arginine (Arg), alanine (Ala), taurine (Tau), anserine (Ans), carnosine (Car), tyrosine (Tyr), valine (Val), methionine (Met), cystine ($(Cys)_2$), tryptophan (Trp), phenylalanine (Phe), isoleucine (Ile), leucine (Leu), lysine (Lys), and proline (Pro). The second sample 136 contains histamine (Him) and at least one of the 24 specific amino acids. The second sample 136 is subjected to pretreatment. The pretreatment performed on the second sample 136 may be the same as the pretreatment performed on the first sample 132.

As the first column 44 and the second column 84, an ODS (Octadecyl silane) column was used. The ODS column is a column in which a filler in which octadecylsilyl groups are chemically bonded to a silica gel carrier is packed. The first column 44 has an inner diameter of 3.0 mm, and the particle size of the particle to be separated is 3 pm. On the other hand, the second column 84 has an inner diameter of 3.0 mm, and the particle size of the particle to be separated is 2.7 pm.

The flow rate of the first mobile phase 54 in the first column 44 was 0.8 mL/min, and the first column 44 was used in a state of being heated to 30°C. In addition, 5 μL of the first sample 132 was injected into the first mobile phase 54. As the first detector 48, a photodiode array detector having a measurement wavelength of 260 nm was used.

The flow rate of the second mobile phase 94 in the second column 84 is 0.8 mL/min, which is the same as the flow rate of the first mobile phase 54 in the first column 44. The second column 84 was used in a state of being heated to 35°C. In addition, 1 μL of the derivatized second sample 137 was injected into the second mobile phase 94. As the second detector 88, a fluorescence detector was used. More specifically, as the fluorescence detector, a multichannel detector having a channel for detecting fluorescence of 450 nm at an excitation wavelength of 350 nm and a channel for detecting fluorescence of 305 nm at an excitation wavelength of 266 nm was used.

The length of the second column 84 is 100 mm. When the first column 44 having a length of 100 mm was used similarly to the second column 84, there was a peak that could not be separated with a sufficient separation degree in the obtained first chromatogram. Therefore, a case where the first column 44 having a length of 100 mm is used will be described below as a comparative example.

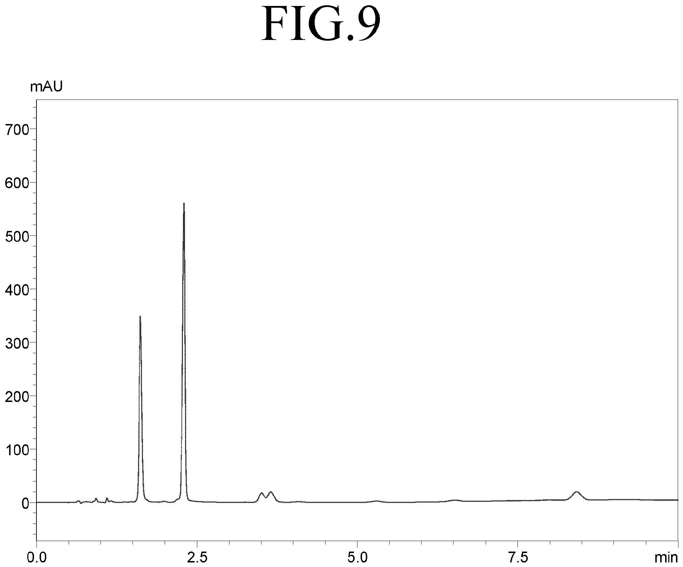

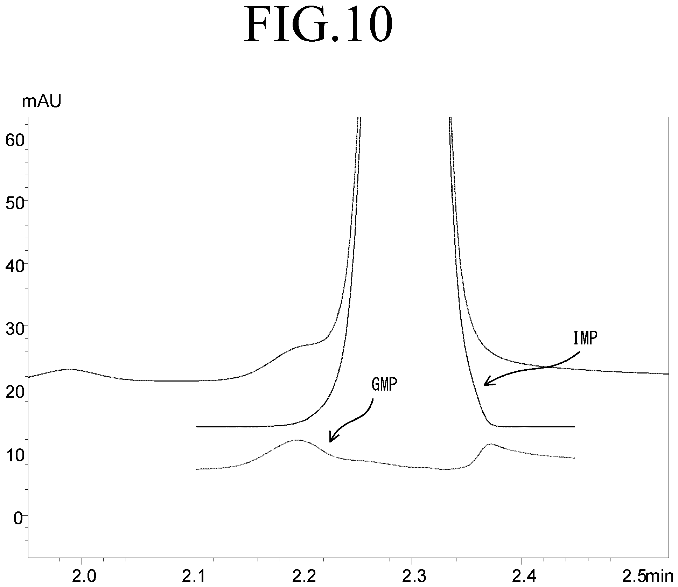

FIG. 9 is a diagram illustrating a first chromatogram in a comparative example. FIG. 10 is an enlarged view of a part of the first chromatogram in FIG. 9.

In FIG. 9, a plurality of peaks corresponding to the ATP-related substance and the like contained in the first sample 132 appear. Among the plurality of peaks, the highest peak appearing in the vicinity of the retention time of 2.3 min is enlarged and illustrated in FIG. 10. This peak can be divided into a peak corresponding to inosinic acid (IMP) and a peak corresponding to guanylic acid (GMP) as additionally illustrated in FIG. 10. That is, in the first chromatogram, since the retention times of inosinic acid and guanylic acid are close, the peak corresponding to inosinic acid and the peak corresponding to guanylic acid cannot be sufficiently separated, and appear as one peak.

In the examples of FIGS. 9 and 10, the separation degree between the peak corresponding to inosinic acid and the peak corresponding to guanylic acid can be calculated using the following Equation (1). In Equation (1), $R$ represents a separation degree, $t_1$ represents a retention time of guanylic acid, $t_2$ represents retention time of inosinic

acid, $W_1$ represents a peak width of guanylic acid, and $W_2$ represents a peak width of inosinic acid.

[Equation 1]

$$R = \frac{t_2 - t_1}{\frac{1}{2}(W_1 + W_2)} \qquad \cdots (1)$$

When the separation degree between the peak corresponding to inosinic acid and the peak corresponding to guanylic acid was calculated using the above Equation (1), the separation degree was 1.1. That is, when a column having a length of 100 mm is used as the first column 44, the peak corresponding to inosinic acid and the peak corresponding to guanylic acid can hardly be separated. In addition, since inosinic acid and guanylic acid have the same detection wavelength, they cannot be separated by the wavelength.

Therefore, in the example, the peak corresponding to inosinic acid and the peak corresponding to guanylic acid are sufficiently separated using a column having a length equal to or longer than a predetermined threshold as the first column 44. That is, the separation degree between the peak corresponding to inosinic acid and the peak corresponding to guanylic acid is set to 1.5 or more.

Hereinafter, the predetermined threshold value related to the length of the first column 44 will be described. When the widths of adjacent peaks are equal, the separation degree is expressed by the following Equation (2). In Equation (2), $R$ is a separation degree, $N$ is a theoretical plate number, $a$ is a separation coefficient, and $k$ is a retention coefficient.

[Equation 2]

$$R = \frac{1}{4}\sqrt{N}\left(\frac{\alpha - 1}{\alpha}\right)\left(\frac{k}{1+k}\right) \qquad \cdots (2)$$

According to the above Equation (2), the separation degree is proportional to the square root of the theoretical plate number. Therefore, if the separation degree is improved from $R$ to $R_1$, that is, if the separation degree is multiplied by $R_1/R$, the theoretical plate number needs to be multiplied by $(R_1{}^2)/(R^2)$.

In addition, since the length of the column is proportional to the theoretical plate number, if the length $L$ of the column corresponding to the separation degree $R$ is multiplied by $(R_1{}^2)/(R^2)$ the length $L_1$ of the column required to obtain the separation degree $R_1$ is calculated. Therefore, the length $L_1$ of the column is expressed by the following Equation (3).

[Equation 3]

$$L_1 = L \times \frac{R_1^2}{R^2} \qquad \cdots (3)$$

As in comparative example described above, when the length of the first column 44 was 100 mm, the separation degree between the peak corresponding to inosinic acid and the peak corresponding to guanylic acid was 1.1 in the first chromatogram obtained using the first column 44. In addition, the separation degree required for sufficiently separating the peak corresponding to inosinic acid and the peak corresponding to guanylic acid is 1.5 or more.

Therefore, in the above Equation (3), when $L$ is 100, $R$ is 1.1, and $R_1$ is 1.5, the length $L_1$ of the first column 44 necessary for obtaining the separation degree 1.5 is calculated to be 185 mm. Therefore, in the example, the length of the first column 44 is 185 mm or more.

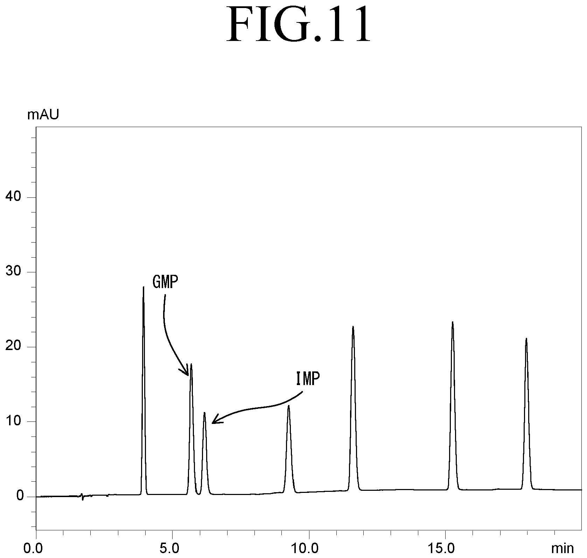

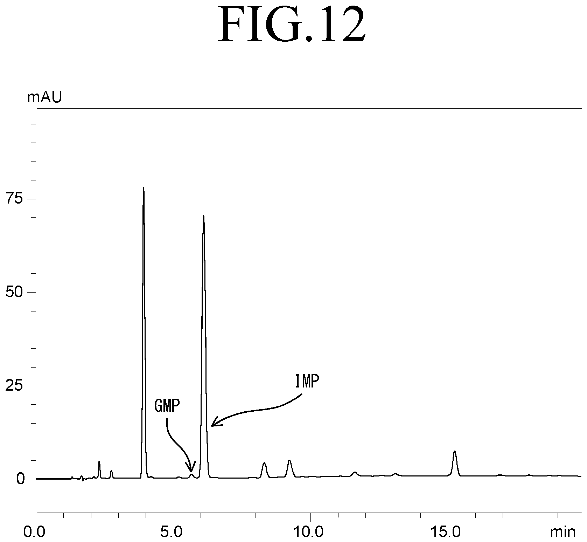

For example, when the length of the first column 44 is 250 mm, the first chromatogram as illustrated in FIGS. 11 and 12 is obtained. FIG. 11 is a diagram illustrating a first chromatogram in example. FIG. 12 is a diagram illustrating another first chromatogram in example. The first chromatogram illustrated in FIG. 11 corresponds to a case where a mixed standard solution is separated by the first column 44, and the first chromatogram illustrated in FIG. 12 corresponds to a case where a sample extracted from tuna is separated by the first column 44. The standard solution herein is a solution obtained by mixing the same components as the components contained in the first sample 132 in the same amounts (50 μmol/L each).

As illustrated in FIG. 11, in the first chromatogram obtained when the mixed standard solution is separated by the first column 44, the peak corresponding to inosinic acid (IMP) and the peak corresponding to guanylic acid (GMP) are completely separated. Also in the first chromatogram using the actual sample illustrated in FIG. 12, the peak corresponding to inosinic acid (IMP) and the peak corresponding to guanylic acid (GMP) are completely separated although the peak heights are different. In the first chromatograms illustrated in FIGS. 11 and 12, the separation degree between the peak corresponding to inosinic acid and the peak corresponding to guanylic acid was 1.9 as calculated using the above Equation (1).

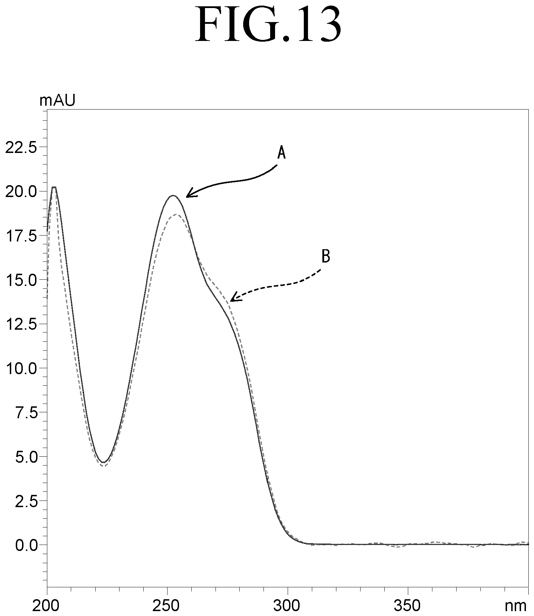

In the first chromatogram illustrated in FIG. 12, the reason why the peak having a retention time of about 5.7 min is a peak corresponding to guanylic acid is illustrated in FIG. 13. FIG. 13 is a diagram illustrating an example of UV spectra in the examples in FIGS. 11 and 12. In FIG. 13, a spectrum Aof a peak corresponding to guanylic acid (GMP) having a retention time of about 5.7 min in the example of FIG. 11 and a spectrum B of a peak having a retention time of about 5.7 min in the example of FIG. 12 are shown on the same graph. As illustrated in FIG. 13, the spectrum A and the spectrum B substantially coincide with each other. That is, in the first chromatogram illustrated in FIG. 11, it is found that a peak having a retention time of about 5.7 min is a peak corresponding to guanylic acid.

In this way, by separating the components contained in the first sample 132 with the first column 44 having a length of 185 mm or more, a first chromatogram in which a peak corresponding to inosinic acid (IMP) and a peak corresponding to guanylic acid (GMP) are separated with a separation degree of 1.5 or more is obtained.

In the above example, a gradient step is performed by controlling the drive of the first pump 26 and the second pump 34 during the first analysis step. That is, the first analysis step includes the gradient step. In the gradient step in the first analysis step, the mixing ratio of the first solution 50 and the second solution 52 is changed to gradually increase the ratio of the organic solvent in the first mobile phase 54.

The first solution 50 is obtained by mixing water and acetonitrile at a volume ratio of 100: 1, and the second solution 52 is obtained by mixing water and acetonitrile at a volume ratio of 80: 20. Both the first solution 50 and the second solution 52 contain 0.15 mol/L of phosphoric acid and 0.225 mol/L of triethylamine.

In the gradient step in the first analysis step, the ratio of the first solution 50 in the first mobile phase 54 is gradually reduced, and the ratio of the second solution 52 is gradually increased. Specifically, only the first solution 50 is introduced as the first mobile phase 54 until 5 minutes elapse from the start of the first analysis step, and thereafter, the ratio of the second solution 52 in the first mobile phase 54 is gradually increased. As a result, when 19 minutes have elapsed from the start of the first analysis step, the ratio of the second solution 52 in the first mobile phase 54 becomes 24%. Since the retention time is long in the first column 44 having a length of 185 mm or more, the first mobile phase 54 can be introduced into the first column 44 by increasing the content ratio of the organic solvent in the second solution 52 and increasing the ratio of the second solution 52 with a high gradient as described above.

Also during the second analysis step performed in parallel with the first analysis step, the gradient step is performed by controlling the drive of the third pump 66 and the fourth pump 74. That is, the second analysis step includes a gradient step. In the gradient step, the mixing ratio of the third solution 90 and the fourth solution 92 is changed to gradually increase the ratio of the organic solvent in the second mobile phase 94.

The third solution 90 is a phosphate buffer having a pH of 6.5 containing 20 mmol/L of phosphate, and the fourth solution 92 is obtained by mixing acetonitrile, methanol, and water at a volume ratio of 45: 40: 15.

In the gradient step in the second analysis step, the ratio of the third solution 90 in the second mobile phase 94 is gradually reduced, and the ratio of the fourth solution 92 is gradually increased. Specifically, until 8 minutes elapse from the start of the second analysis step, the ratio of the fourth solution 92 in the second mobile phase 94 is gradually increased from 5% to 13%. Thereafter, the ratio of the fourth solution 92 in the second mobile phase 94 is gradually increased from 13% to 25% until 15 minutes elapse from the start of the second analysis step. Thereafter, the ratio of the fourth solution 92 in the second mobile phase 94 is gradually increased from 25% to 52% until 21.5 minutes elapse from the start of the second analysis step.

According to the gradient step, the time until the elution of each component is completed can be shortened without deteriorating the separation degree at the peak of the component having a short retention time among the components contained in the first sample 132 or the derivatized second sample 137. That is, it is possible to suppress the time until the elution of each component contained in the first sample 132 or the derivatized second sample 137 is completed from becoming longer as the length of the first column 44 or the second column 84 becomes longer.

Further, the first analysis step and the second analysis step include a washing step. The washing step is performed after the gradient step.

The washing step in the first analysis step is a step of introducing only the second solution 52 as the first mobile phase 54 into the first column 44 and washing the inside of the first column 44 for 19.10 min to 25.00 min from the start of the first analysis step. Thereafter, only the first solution 50 is introduced into the first column 44 as the first mobile phase 54 for 25.10 min to 32.00 min from the start of the first analysis step.

The washing step in the second analysis step is a step of introducing only the fourth solution 92 as the second mobile phase 94 into the second column 84 and washing the inside of the second column 84 for 21.51 min to 27.50 min from the start of the second analysis step. Thereafter, only the third solution 90 is introduced into the second column 84 as the second mobile phase 94 for 27.51 min to 32.00 min from the start of the second analysis step.

According to the washing step, since contaminants are not accumulated in the first column 44 or the second column 84, it is possible to suppress an increase in load on the first column 44 or the second column 84. That is, deterioration of the first column 44 or the second column 84 can be suppressed.

In such a liquid chromatograph 10, as described above, since the introduction of the first mobile phase 54 into the first column 44 and the introduction of the second mobile phase 94 into the second column 84 are continued until both the first analysis step and the second analysis step are completed, even if each component contained in the first sample 132 is already detected by the first detector 48, the first mobile phase 54 is continuously introduced into the first column 44 until each component contained in the derivatized second sample 137 is detected by the second detector 88. In the first analysis step, each component contained in the first sample 132 is detected by the first detector 48 until 19 minutes elapse from the start of the first analysis step.

As described above, since the analysis of each component contained in the first sample 132 is completed before the analysis of each component contained in the derivatized second sample 137 is completed, the first column 44 having a length of 185 mm or more can be sufficiently washed by performing the washing step during the first analysis step over a relatively long time before both the first analysis step and the second analysis step are completed.

5. Specific electrical configuration of liquid chromatograph

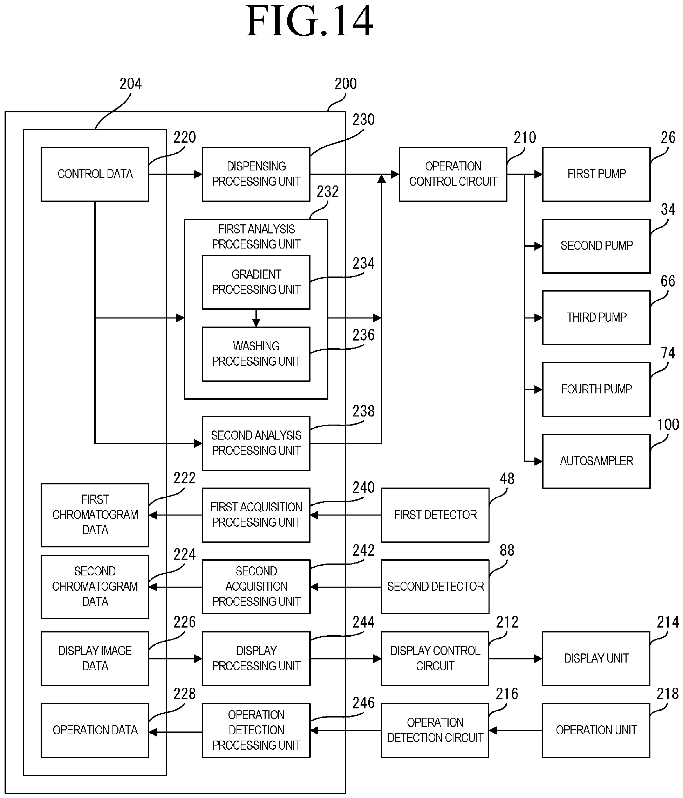

FIG. 14 is a block diagram specifically illustrating an electrical configuration of the liquid chromatograph 10 of the present embodiment. In FIG. 14, the storage unit 206 is not illustrated.

The RAM 204 stores execution data read in advance from the storage unit 206. In addition, when acquired data acquired using a device, a sensor, or the like is stored in the storage unit 206, the acquired data is temporarily stored in the RAM 204.

In the example illustrated in FIG. 14, the RAM 204 stores control data 220, first chromatogram data 222, second chromatogram data 224, display image data 226, and operation data 228.

The control data 220 is various data necessary for controlling the operation in the liquid chromatograph 10. The first chromatogram data 222 is data corresponding to the first chromatogram. The second chromatogram data 224 is data corresponding to the second chromatogram. The display image data 226 is data corresponding to the display image. The operation data 228 is data representing an operation state of the operation unit 218.

Although not illustrated, a control program read in advance from the storage unit 206 is stored in the RAM 204, and when the CPU 202 executes the control program, the control unit 200 functions as a dispensing processing unit 230, a first analysis processing unit 232, a second analysis processing unit 238, a first acquisition processing unit 240, a second acquisition processing unit 242, a display processing unit 244, and an operation detection processing unit 246. The first analysis processing unit 232 includes a gradient processing unit 234 and a washing processing unit 236. Although not illustrated, the second analysis processing unit 238 also includes a gradient processing unit and a washing processing unit.

The dispensing processing unit 230 controls the autosampler 100 using the control data 220 to perform the dispensing step. The first analysis processing unit 232 performs the first analysis step by controlling the first pump 26, the second pump 34, and the autosampler 100 using the control data 220.

The gradient processing unit 234 performs a gradient step by controlling the first pump 26 and the second pump 34 using the control data 220. After the gradient step is performed, the washing processing unit 236 controls the second pump 34 using the control data 220 to perform the washing step.

The second analysis processing unit 238 controls the third pump 66, the fourth pump 74, and the autosampler 100 to perform the second analysis step.

The first acquisition processing unit 240 acquires the first chromatogram data 222 based on the signal from the first detector 48 and stores the first chromatogram data 222 in the RAM 204. The second acquisition processing unit 242 acquires the second chromatogram data 224 based on the signal from the second detector 88 and stores the second chromatogram data in the RAM 204.

The display processing unit 244 outputs the display image data 226 to the display unit 214. The operation detection processing unit 246 detects an operation on the operation unit 218 and stores the operation data 228 in the RAM 204 in chronological order.

6. Aspects

It is understood by those skilled in the art that the plurality of exemplary embodiments described above are specific examples of the following aspects.

(Aspect 1) An analytical method according to one aspect is an analytical method for performing, in parallel, a first analysis step of introducing a non-derivatized first sample into a first column together with a first mobile phase, and analyzing components contained in the first sample, and a second analysis step of introducing a derivatized second sample into the second column together with a second mobile phase, and analyzing components contained in the second sample, using a first column and a second column each constituted by an ODS column, wherein the first sample may contain inosinic acid and guanylic acid, and in the first analysis step, by separating components contained in the first sample using the first column having a length of 185 mm or more, a chromatogram may be obtained in which peaks corresponding to inosinic acid and guanylic acid are separated with a separation degree of 1.5 or more.

According to the analytical method described in Aspect 1, it is possible to acquire a chromatogram in which peaks corresponding to inosinic acid and guanylic acid contained in the first sample are sufficiently separated while analyzing components contained in each of the non-derivatized first sample and the derivatized second sample in parallel. That is, the peak corresponding to guanylic acid, which is an example of a flavor component contained in food, can be separated from the peak corresponding to inosinic acid without separately providing a column.

(Aspect 2) The analytical method according to Aspect 1, wherein
the first analysis step may include a gradient step of gradually increasing a ratio of an organic solvent in the first mobile phase to be introduced into the first column by changing a mixing ratio between the first solution and the second solution having a higher organic solvent content ratio than that of the first solution.

According to the analytical method described in Aspect 2, it is possible to shorten the time until the elution of each component is completed without deteriorating the separation degree at the peak of the component having a short retention time among the components contained in the first sample. That is, it is possible to suppress the time until the elution of each component contained in the first sample is completed from becoming longer as the length of the first column becomes longer.

(Aspect 3) The analytical method according to Aspect 2, wherein
the first analysis step may include a washing step of washing the inside of the first column by introducing only the second solution into the first column after the gradient step.

According to the analytical method described in Aspect 3, since the contaminants are not accumulated in the first column, it is possible to suppress an increase in load on the first column. That is, deterioration of the first column can be suppressed.

In addition, if the analysis of each component contained in the first sample is completed before the analysis of each component contained in the derivatized second sample is completed, the first column can be sufficiently washed by performing the washing step during the first analysis step over a relatively long time before both the first analysis step and the second analysis step are completed.

DESCRIPTION OF REFERENCE SIGNS

| | |
|---|---|
| 44 | first column |
| 50 | first solution |
| 52 | second solution |
| 54 | first mobile phase |
| 84 | second column |
| 94 | second mobile phase |
| 132 | first sample |
| 136 | second sample |
| 137 | derivatized second sample |
| 222 | first chromatogram data |
| 232 | first analysis processing unit |
| 234 | gradient processing unit |
| 236 | washing processing unit |
| 238 | second analysis processing unit |
| 240 | first acquisition processing unit |

**Claims**

1. An analytical method for performing, in parallel:

   a first analysis step of introducing a non-derivatized first sample into a first column together with a first mobile phase, and analyzing components contained in the first sample; and
   a second analysis step of introducing a derivatized second sample into a second column together with a second mobile phase, and analyzing components contained in the second sample, using the first column and the second column each constituted by an ODS column,
   wherein the first sample contains inosinic acid and guanylic acid, and
   in the first analysis step, by separating components contained in the first sample using the first column having a length of 185 mm or more, a chromatogram is obtained in which peaks corresponding to inosinic acid and guanylic acid are separated with a separation degree of 1.5 or more.

2. The analytical method according to claim 1, wherein the first analysis step includes a gradient step of gradually increasing a ratio of an organic solvent in the first mobile phase to be introduced into the first column by changing a mixing ratio between a first solution and a second solution having a higher organic solvent content ratio than that of the first solution.

3. The analytical method according to claim 2, wherein the first analysis step includes a washing step of washing an inside of the first column by introducing only the second solution into the first column after the gradient step.

# FIG.1

10

FIG.2

# FIG.3

# FIG.4

# FIG.5

# FIG.6

# FIG.7

# FIG.8

# FIG.9

# FIG.10

# FIG.11

# FIG.12

# FIG.13

# FIG.14

## INTERNATIONAL SEARCH REPORT

International application No.

**PCT/JP2022/004043**

### A. CLASSIFICATION OF SUBJECT MATTER

***B01J 20/281***(2006.01)i; ***B01J 20/283***(2006.01)i; ***G01N 30/06***(2006.01)i; ***G01N 30/34***(2006.01)i; ***G01N 30/50***(2006.01)i; ***G01N 30/86***(2006.01)i; ***G01N 30/88***(2006.01)i

FI: G01N30/88 F; B01J20/281 X; B01J20/283; G01N30/06 E; G01N30/34 E; G01N30/86 E; G01N30/50

According to International Patent Classification (IPC) or to both national classification and IPC

### B. FIELDS SEARCHED

Minimum documentation searched (classification system followed by classification symbols)

B01J20/281; B01J20/283; G01N30/06; G01N30/34; G01N30/50; G01N30/86; G01N30/88

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

Science Direct

### C. DOCUMENTS CONSIDERED TO BE RELEVANT

| Category* | Citation of document, with indication, where appropriate, of the relevant passages | Relevant to claim No. |
|---|---|---|
| Y | WO 2020/183663 A1 (SHIMADZU CORP) 17 September 2020 (2020-09-17)<br>    claim 1 | 1-3 |
| Y | 吉川秀樹　ほか, 市販うどんつゆの呈味成分の比較, 京都光華女子大学研究概要, December 2012, 50, pp. 123-128<br>    p. 2, left column, 6. Analysis of free amino acids, (YOSHIKAWA, Hideki et al. Comparative Studies on the Taste Components of Commercial Udon Soup. Research bulletin of Kyoto Koka Women's University.) | 1-3 |
| Y | JP 61-254852 A (SHIMADZU CORP) 12 November 1986 (1986-11-12)<br>    claim 1 | 1-3 |
| Y | JP 63-232846 A (OGURA, Haruo) 28 September 1988 (1988-09-28)<br>    p. 5, lower right column, example 3 | 3 |

☐ Further documents are listed in the continuation of Box C.    ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **12 April 2022** | **26 April 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2022/004043**

| Patent document cited in search report | | | Publication date (day/month/year) | Patent family member(s) | Publication date (day/month/year) |
|---|---|---|---|---|---|
| WO | 2020/183663 | A1 | 17 September 2020 | (Family: none) | |
| JP | 61-254852 | A | 12 November 1986 | (Family: none) | |
| JP | 63-232846 | A | 28 September 1988 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020183663 A